Europäisches Patentamt

European Patent Office

Office européen des brevets

(19)

(11) Veröffentlichungsnummer: **0 116 610**
**B1**

(12) # EUROPÄISCHE PATENTSCHRIFT

(45) Veröffentlichungstag der Patentschrift:
**19.11.87**

(21) Anmeldenummer: **83902756.2**

(22) Anmeldetag: **19.08.83**

(86) Internationale Anmeldenummer:
**PCT/EP 83/00223**

(87) Internationale Veröffentlichungsnummer:
**WO 84/00896 (15.03.84** Gazette **84/7)**

(51) Int. Cl.⁴: **A 61 N 1/16,** G 01 V 3/00,
H 01 Q 1/00

(54) **STRAHLUNGS-ABFANGVORRICHTUNG.**

(30) Priorität: **27.08.82 DE 3232043**

(43) Veröffentlichungstag der Anmeldung:
**29.08.84 Patentblatt 84/35**

(45) Bekanntmachung des Hinweises auf die Patenterteilung:
**19.11.87 Patentblatt 87/47**

(84) Benannte Vertragsstaaten:
**AT CH FR GB LI NL**

(56) Entgegenhaltungen:
**DE - A - 3 027 367**
**FR - A - 1 104 305**
**FR - A - 1 231 621**
**FR - A - 1 604 700**

(73) Patentinhaber: **MICHAEL GEISELER
DBF-VERTRIEBS-GMBH, Arabellastrasse 5,
D-8000 Munchen 81 (DE)**

(72) Erfinder: **OBERBACH, Josef, Schlosstrasse 9,
D-8022 Grünwald (DE)**

(74) Vertreter: **Wagner, Karl H. et al, WAGNER & GEYER
Patentanwälte Gewuerzmuehlstrasse 5 Postfach 246,
D-8000 München 22 (DE)**

ACTORUM AG

## Beschreibung

Die Erfindung bezieht sich auf eine Vorrichtung gemäss dem Oberbegriff des Anspruchs 1.

Elektromagnetische Strahlung tritt insbesondere auch in einer die Gesundheit der Menschen schädigenden Weise bei Fernsehgeräten und elektronisch arbeitenden Spielgeräten, Maschinen und anderen Einrichtungen auf. Als ein weiteres Beispiel seien ferner die in Banken, Verwaltungen, Industriebetrieben und Rechenzentren benutzten Computer genannt. Die genannte Strahlung kann auch in Praxen, Kopieranstalten und Haushalten durch die verwendeten elektrischen Geräte hervorgerufen werden. Darüberhinaus tritt die Strahlung auch bei Radargeräten auf Flugplätzen und Schiffen, Sendern für Rundfunk und Fernsehen sowie bei Mikrowellengeräten in Hotels und Haushaltungen auf. Gleiches gilt für Verstärkeranlagen, elektronische Orgeln und Rekorder sowie Uhren.

Es ist bereits aus der DE-OS 30 27 368 bekannt, zum Einfangen von Strahlungen einen langgestreckten Hohlraum vorzusehen, der in geeigneter Weise angeordnet und mit einem Ableiter ausgestattet ist, der seinerseits mit der Erdung in Verbindung steht.

Aus der FR-A-1 104 305 ist eine Vorrichtung zum Einfangen und Ableiten von schädlichen Strahlungen bekannt, die gemäss dem Oberbegriff des Anspruchs 1 ausgebildet ist. Der vorliegenden Erfindung liegt die Erkenntnis zugrunde, dass die Effektivität der bekannten Vorrichtung dadurch beträchtlich erhöht werden kann, dass man an dem Grundprinzip verschiedene Weiterentwicklungen vorsieht. So ist gemäss der Erfindung eine Ausgestaltung entsprechend dem kennzeichnenden Teil des Anspruchs 1 vorgesehen.

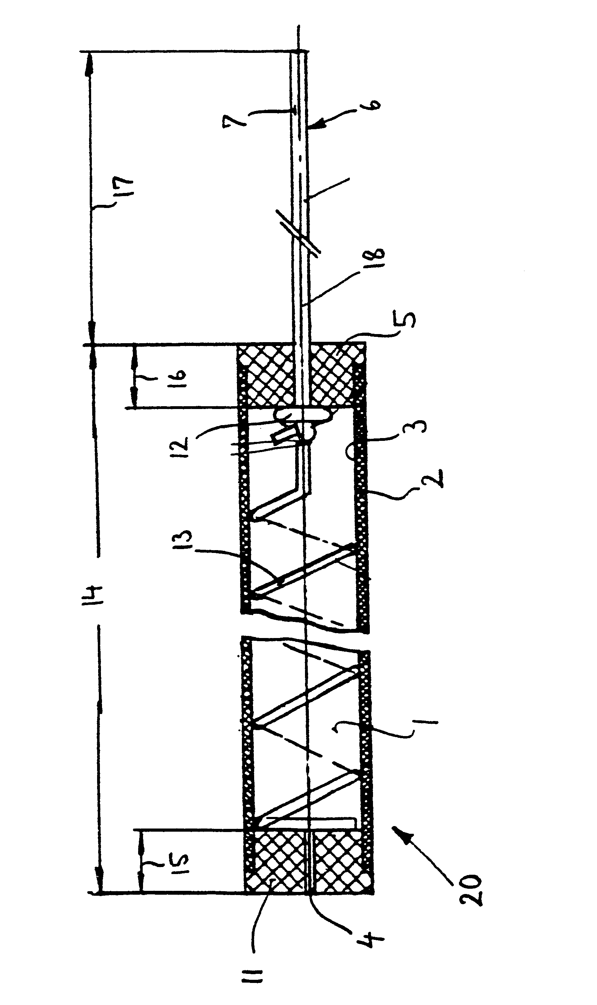

Bevorzugte weitere Ausgestaltungen der Erfindung ergeben sich insbesondere aus der folgenden Beschreibung eines Ausführungsbeispiels sowie den Patentansprüchen. In der Zeichnung ist die erfindungsgemässe Vorrichtung im Schnitt dargestellt.

Die Vorrichtung 20 dient zum Empfangen elektromagnetischer Strahlung und zu deren Ableitung gegen die Erde. Die erfindungsgemässe Strahlungs-Abfangvorrichtung 20 weist einen langgestreckten durch eine Hülse 2 gebildeten Hohlraum 1 auf. Die Hülse 2 ist an ihren beiden an sich offenen Enden jeweils durch einen Stopfen 5, bzw. 11 abgeschlossen. Die Mittelachse der Hülse 2 ist mit dem Bezugszeichen 18 versehen. Der Stopfen 11 besitzt eine auf der Achse 18 verlaufende Öffnung 4. Auch der Stopfen 5 ist mit einer nicht näher bezeichneten Öffnung ausgestattet, die ebenfalls auf der Achse 18 verläuft und den Durchtritt eines Ableiters 6 gestattet. Der Ableiter 6 besteht aus einer Kupferlitze, die mit einer Isolierschicht 7 überzogen ist. Die mit 17 bezeichnete Länge des Ableiters 6 ist derart vorgesehen, dass das in der Figur rechts gelegene Ende mit der Erdung verbunden sein kann. Das in der Figur links gelegene Ende des Ableiters 6 bildet einen Knoten 12 und liegt auf der Innenseite des Stopfens 5 auf. Zusätzlich kann der Ableiter 6 noch zum Zwecke einer Halterung im Stopfen 5 festgeklebt sein. Im Hohlraum 1 erstreckt sich ein aus isoliertem Kupferdraht bestehender Leiter 13, der, wie gezeigt, schraubenlinienförmig gewickelt ist. Vorzugsweise liegt der Leiter 13 mit seinen Windungen an der Innenwand der Hülse 2 an. Der schraubenlinienförmige gewendelte Leiter 13 liegt mit seinem – in der Zeichnung – linken Ende auf der Innenseite des Stopfens 11 in der gezeigten Weise auf und kann beispielsweise an der Innenseite des Stopfens 11 festgeklebt sein. Vorzugsweise bildet der Leiter 13 sieben Windungen. Auch Vielfache der Windungszahl 7 können vorgesehen sein.

Der Leiter 13 endet benachbart zum Stopfen 5 mit einem kurzen, im wesentlichen auf der Achse 18 verlaufenden Endteil. Dieser Endteil liegt unmittelbar am Knoten 12 an.

Die Hülse 2 besteht vorzugsweise aus Kunststoff. Der Vorteil davon ist, dass sich ein geringes Gewicht bei gleichzeitiger Unzerbrechlichkeit ergibt. Vorzugsweise ist die Länge der Hülse etwa 12,5 cm. Die Gesamtlänge 14 der Strahlungs-Abfangvorrichtung 20 beträgt vorzugsweise 14 cm oder ein Vielfaches davon. Auch eine Länge 14 von 7 cm ist möglich. Die Längen 15, 16 der Stopfen 4, 5 sind vorzugsweise 7,5 mm.

Der Aussendurchmesser der Hülse 2 beträgt vorzugsweise 1,5 cm. Der Innendurchmesser der Hülse 2 beträgt ungefähr 1,4 mm.

Vorzugsweise bestehen die beiden Stopfen 5 und 11 aus folgendem Material: Kunststoff. Der Leiter B ist entweder rechts oder links herum gewendelt.

## Patentansprüche

Vorrichtung zum Empfangen und Ableiten von elektromagnetischen Strahlungen, die an elektrischen Geräten auftreten, bestehend aus einer langgestreckten Hülse (2), in der ein schraubenlinienförmig gewendelter Leiter (13) aus isoliertem Kupferdraht angeordnet ist, welcher sich über die gesamte Länge des von der Hülse umschlossenen Hohlraumes (1) erstreckt, dadurch gekennzeichnet, dass die Hülse an beiden Enden durch Stopfen (5; 11) abgeschlossen ist, von denen der eine eine Öffnung (4) aufweist und der andere eine Austrittsöffnung, aus der zum Zwecke der Erdung ein Ableiter (6) herausragt, welcher aus einer mit einer Isolierschicht (7) überzogenen Kupferlitze besteht, und dass der Ableiter (6) im Hohlraum (1) anliegend an der Innenwand des Stopfens (5) einen Knoten (12) aufweist, an dem ein auf der Achse der Hülse verlaufender kurzer Endteil der Leiterwendel anliegt.

2. Vorrichtung nach Anspruch 1, dadurch gekennzeichnet, dass der Leiter (13) sieben Windungen besitzt.

3. Vorrichtung nach Anspruch 1 oder 2, dadurch gekennzeichnet, dass die Hülse (2) aus Kunststoff besteht.

4. Vorrichtung nach einem oder mehreren der vorhergehenden Ansprüche, dadurch gekennzeichnet, dass die Stopfen (5, 11) aus Kunststoff bestehen und in die Hülsenenden eingeklebt sind.

## Claims

1. An apparatus for receiving and removing electromagnetic radiation occuring at electrical devices, said apparatus comprising an elongate sleeve (2), a conductor (13) made of an insulated copper wire wound in a spiral-shaped manner and arranged within said sleeve, said conductor extending along the entire length of the cavity (1) and closed by said sleeve, characterized in that the sleeve is closed at both ends by means of plugs (5; 11), one of said plugs comprising an opening (4) while the other one comprises an exit opening, wherein a removing wire (6) extends out of said exit opening for the purpose of grounding, said removing wire consisting of a copper wire arrangement being coated with an insulating layer (7), and that the removing wire (6) comprises a knot (12) adjacent to the inner wall of the plug, a short end portion of said spiral-shaped conductor arranged on the axis of said sleeve being in abutment with said knot.

2. The apparatus of claim 1, characterized in that the conductor (13) comprises seven windings.

3. The apparatus of claim 1 or 2, characterized in that the sleeve (2) is made of a plastic material.

4. The apparatus of one or more of the preceding claims, characterized in that the plugs (5; 11) consist of plastic material and are glued to the ends of the sleeve.

## Revendications

1. Dispositif pour capter et évacuer des rayonnements électromagnétiques qui se manifestent dans des appareils électriques, dispositif composé d'un manchon allongé (2) dans lequel est disposé un conducteur (13) en fil de cuivre isolé, enroulé sous une forme hélicoïdale et s'étendant sur toute la longueur de la cavité (1) entourée par le manchon, caractérisé en ce que le manchon est obturé, à ses deux extrémités, par des bouchons (5; 11), dont l'un présente un orifice (4) et l'autre une ouverture de sortie par laquelle s'extrait, en vue de la mise à la terre, un conducteur de sortie (6) qui est constitué par une tresse en cuivre revêtue d'une couche isolante (7), et en ce que le conducteur de sortie (6) comporte un nœud (12) qui s'applique contre la paroi intérieure du bouchon (5), dans la cavité (1), et au contact duquel est placé un court tronçon d'extrémité de l'enroulement conducteur, s'étendant sur l'axe du manchon.

2. Dispositif selon la revendication 1, caractérisé en ce que le conducteur (13) possède sept spires.

3. Dispositif selon la revendication 1 ou 2, caractérisé en ce que le manchon (2) est en matière plastique.

4. Dispositif selon une ou plusieurs des revendications précédentes, caractérisé en ce que les bouchons (5, 11) sont en matière plastique et sont collés dans les extrémités du manchon.